# EUROPÄISCHE PATENTSCHRIFT

(11) **EP 1 896 389 B1**
(45) Veröffentlichungstag und Bekanntmachung des Hinweises auf die Patenterteilung: **23.09.2015**
(21) Anmeldenummer: 06754056.7
(22) Anmeldetag: 01.06.2006
(51) Int. Cl.: C07C 37/14, C07C 39/23, C07D 311/80

(54) **VERFAHREN ZUR HERSTELLUNG VON DRONABINOL AUS CANNABIDIOL UNTER VERWENDUNG EINES MOLEKULARSIEBS**
METHOD FOR THE PRODUCTION OF DRONABINOL FROM CANNABIDIOL, USING A MOLECULAR SIEVE
PROCEDE DE PRODUCTION DE DRONABINOL A PARTIR DE CANNABIDIOL AU MOYEN D'UN TAMIS MOLECULAIRE

(30) Priorität: 22.06.2005 DE 102005028937
(43) Veröffentlichungstag der Anmeldung: 12.03.2008
(73) Patentinhaber: Bionorica Ethics GmbH, 92318 Neumarkt i. d. Opf. (DE)
(72) Erfinder: ERLER, Joachim, 91056 Erlangen (DE); HEITNER, Stefan, 90763 Fürth (DE)
(74) Vertreter: Simandi, Claus
(86) Internationale Anmeldenummer: PCT/EP2006/005250
(87) Internationale Veröffentlichungsnummer: WO 2006/136273

(56) Entgegenhaltungen:
- DE-A1- 4 100 441
- DE-C1- 10 051 427
- US-A- 4 025 516
- US-A- 5 342 971

## Beschreibung

Die vorliegende Erfindung betrifft ein Verfahren zur Herstellung von Dronabinol ((6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹-THC)) aus Cannabidiol (CBD) durch Cyclisierung von Cannabidiol (CBD) (2-[1R-3-Methyl-6-(1-methylethenyl)-2-cyclohexen-1-yl]-5-pentyl-1,3-benzoldiol) zu Δ⁹-THC gemäß dem Oberbegriff des Anspruchs 1.

Cannabis (Hanf) gehört zusammen mit der Gattung Humulus (Hopfen) der Familie der Cannabidaceae an, wobei z. B. Hopfen keine Cannabinoide enthält. Für die botanische und chemotaxonomische Differenzierung der Gattung Cannabis existieren zwei unterschiedliche Konzepte. Man unterscheidet drei Arten Cannabis sativa Linnaeus, Cannabis indica LAM. und Cannabis ruderalis, während eine andere Lehrmeinung nur die eine Sammelart Cannabis sativa L. aus den Unterarten Cannabis sativa ssp. sativa und ssp. indica bestehend sieht. Ferner wird die Cannabispflanze in einen Drogen- und einen Fasertyp differenziert, wobei die Differenzierung aufgrund des mengenmäßigen Verhältnisses der Hauptcannabinoide Cannabidiol (CBD) und Δ⁹-Tetrahydrocannabinol (Δ⁹-THC) geschieht. Faserhanf, dessen Anbau zur Fasergewinnung zugelassen ist, darf einen Δ⁹-THC-Gehalt von 0,3%, bezogen auf die Pflanzentrockenmasse, nicht überschreiten, während der Drogentyp einen Δ⁹-THC-Gehalt von ca. 5% - 15%, bezogen auf die Pflanzentrockenmasse, aufweisen kann.

Die bekannten, halluzinogen wirkenden Cannabispräparate Marihuana und Haschisch unterliegen in Deutschland wie Heroin, Kokain und LSD als nicht verkehrsfähige Betäubungsmittel den Bestimmungen des Betäubungsmittelgesetzes.

Cannabis sativa L. enthält über 420 verschiedene Inhaltsstoffe, davon gehören 61 Verbindungen der Klasse der Cannabinoide an. Es handelt sich hierbei um lipophile, stickstofffreie, meist phenolische Verbindungen. Die neutralen Cannabinoide sind biogenetisch aus einem Monoterpen und einem Phenol, die sauren Cannabinoide aus einem Monoterpen und einer Phenolcarbonsäure abgeleitet und weisen einen C₂₁-Grundkörper auf. In der Literatur sind zwei unterschiedliche Nummerierungssysteme für Cannabinoide zu finden. Das ältere Nummerierungssystem basiert auf dem Monoterpen-Grundgerüst, während die neuere IUPAC-Bezeichnung, die ausschließlich in der vorliegenden Anmeldung verwendet wird, sich auf das Dibenzopyran-Grundgerüst bezieht.

Die in dieser Anmeldung verwendete Nummerierung der Ringatome für beispielsweise Δ⁹-Tetrahydrocannabinol (Δ⁹-THC) ist demnach wie folgt:

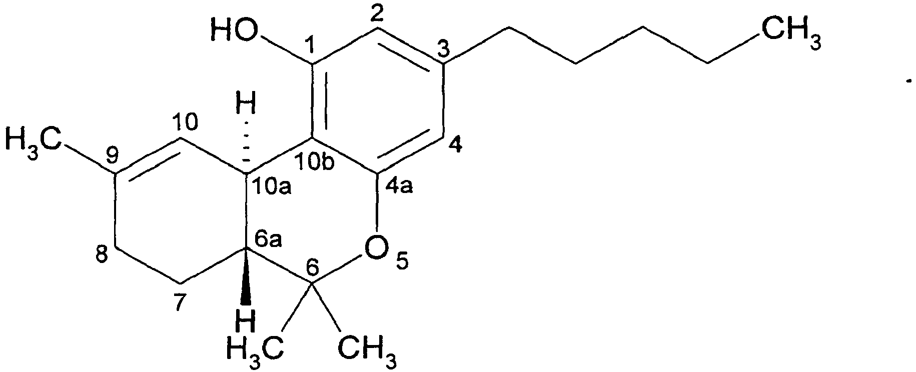

Zu den wichtigsten Cannabinoiden gehören:

| | |
|---|---|
| Δ⁹-Tetrahydrocannabinol | Δ⁹-THC |
| Δ⁸-Tetrahydrocannabinol | Δ⁸-THC |
| Cannabichromen | CBC |
| Cannabidiol | CBD |
| Cannabigerol | CBG |
| Cannabinidiol | CBND |
| Cannabinol | CBN |

Neben den oben erwähnten Cannabinoiden finden sich noch deren zugehörige Carbonsäuren in der Rohdroge sowie in den Pflanzenprodukten.

In der Regel haben die Carbonsäuren die Funktion eines biosynthetischen Precursors. So entstehen beispielsweise in vivo aus den THC-Carbonsäuren durch Decarboxylierung die Tetrahydrocannabinole Δ⁹- und Δ⁸-THC und CBD aus den zugehörigen Cannabidiolcarbonsäuren.

Δ⁸-THC kann beispielsweise auch beim Ringschluß von CBD entstehen. Eine andere Möglichkeit liegt darin, daß unter bestimmten Bedingungen beispielsweise durch Säure, Δ⁸-THC durch Doppelbindungsisomerie aus Δ⁹-THC beziehungsweise dessen Carbonsäure entstehen kann.

Im folgenden sind die chemischen Strukturen einiger Cannabinoid-Wirkstoffe und die Nomenklatur der beiden Wirkstoffe des Tetrahydrocannabinols, deren IUPAC Namen (6aR-trans)-6a,7,8,10a-tetrahydro-6,6,9-trimethyl-3-pentyl-6H-dibenzo[b,d]pyran-1-ol oder Δ⁹-THC und (6aR-trans)-6a,7,10,10a-tetrahydro-6,6,9-trimethyl-3-pentyl-6H-dibenzo[b,d]pyran-1-ol oder Δ⁸-THC sind, angegeben. Δ⁹-THC ist auch unter dem Namen Dronabinol bekannt.

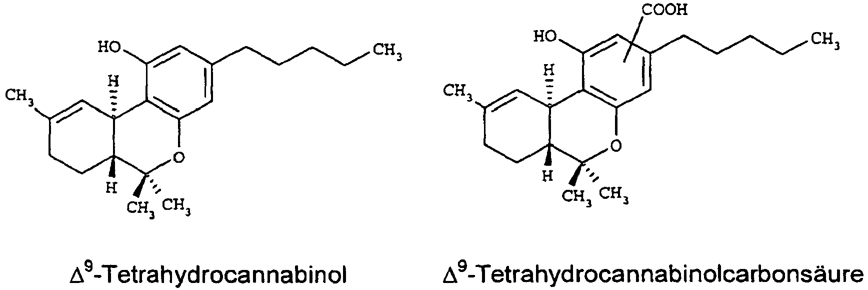

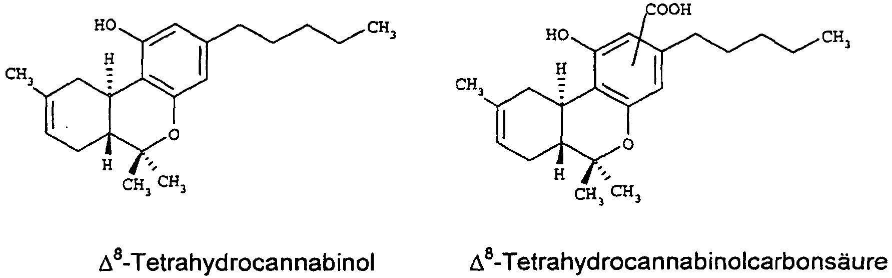

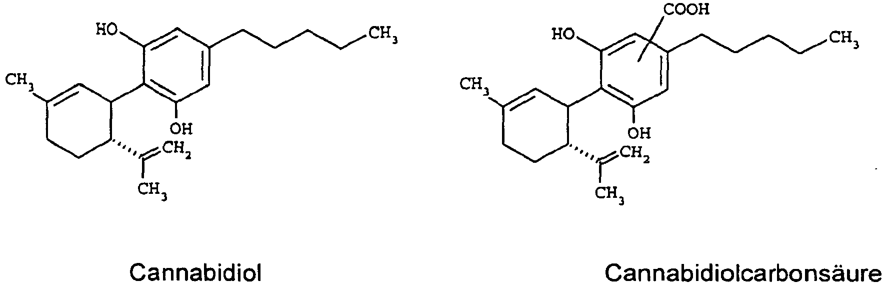

Für die Zwecke der vorliegenden Erfindung soll der Begriff "Tetrahydrocannabinol" oder "THC" - sofern nichts anderes bezeichnet ist - sämtliche Isomere, insbesondere Doppelbindungsisomere, umfassen.

In vielen Kulturkreisen und seit langer Zeit ist Cannabis eine traditionelle Droge sowie ein Heilmittel. Bis in das 20. Jahrhundert hinein wurde Cannabis bei den verschiedensten Beschwerden - vom Asthma bis zur Migräne - eingesetzt. Eine restriktive Gesetzgebung gegen Cannabis seitens der USA führte jedoch schließlich zu einem völligen Verschwinden aus den Arzneibüchern und aus dem Behandlungsrepertoire der Ärzte.

Mittlerweile finden in der klinischen Forschung viele der überlieferten therapeutischen Effekte Bestätigung. Heute ist der pharmakologische Einsatz von Cannabiswirkstoffen im wesentlichen von Bedeutung bei folgenden Indikationen:
- die appetitanregende Wirkung insbesondere bei Aids-Erkrankungen, die mit Kachexie und Wasting-Syndrom einhergehen,
- die antiemetische Wirkung zur Hemmung von Übelkeit und Erbrechen, vor allem im Zusammenhang mit einer Chemotherapie unter Zytostatikagabe,
- die Reduzierung muskulärer Krämpfe und Spastiken bei Multipler Sklerose und Querschnittslähmungen,
- die Schmerz- und Migränebehandlung - bei chronischer Schmerztherapie auch ergänzend zur Opioidbehandlung,
- die Senkung des Augeninnendrucks beim Glaukom,
- die Stimmungsaufhellung,
sowie insbesondere Cannabidiol als Antiepileptikum

Aufgrund des interessanten therapeutischen Spektrums der Cannabinoide wurde eine Reihe von Versuchen unternommen, die Cannabinoide anzureichern, zu isolieren und/oder zu synthetisieren.

So offenbart beispielsweise die DE 10051427 der Anmelderin, auf die hiermit vollinhaltlich verwiesen wird, ein Verfahren zur Herstellung eines Tetrahydrocannabinol- und Cannabidiol-haltigen Extraktes aus Industriehanf sowie Cannabis-Extrakte.

Die DE 41 00 441 A1 beschreibt ein Verfahren zur Herstellung von 6,12-Dihydro-6-hydroxy-Cannabidiol und dessen Verwendung zur Herstellung von trans-Δ⁹-Tetrahydrocannabinol. Insbesondere beschreibt die DE 41 00 441 A1 die Herstellung von 6,12-Dihydro-6-hydroxy-Cannabidiol, das durch Umsetzen von Olivetol und cis-p-Menth-2-en-1,8-diol erhalten wird und dessen weitere Umsetzung zu trans-Δ⁹-Tetrahydrocannabinol unter dem Einsatz geeigneter Katalysatoren.

Mittlerweile ist Dronabinol, Δ⁹-THC in USA gemäß USP 24, S. 613 und 614 als Medikament - auch in Kapselform - zugelassen. Gemäß dieser Monographie enthält Dronabinol nicht weniger als 95% Δ⁹-THC und nicht mehr als 2% Δ⁸-THC.

Seit dem 1. Februar 1998 ist Dronabinol in Deutschland als Betäubungsmittel verschreibungsfähig.

Bei der Herstellung von Dronabinol ((6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹-THC)) ist die Cyclisierung von Cannabidiol (CBD) (2-[1R-3-Methyl-6-(1-methylethenyl)-2-cyclohexen-1-yl]-5-pentyl-1,3-benzoldiol) zu Δ⁹-THC der entscheidende chemische Schritt.

Für diese Cyclisierung sind mehrere Synthesewege bekannt. Neben der klassischen säurekatalysierten Synthese von Petrzilka et al. (Synthese von Haschisch-Inhaltsstoffen, Helvetica Chimica Acta, Vol. 52, Fasc. 4, Nr. 123, pp 1102-1133 (1969)) existiert noch das Verfahren mittels Lewis-Säure als Katalysator. In den folgenden US-Patenten (Nr. 4 025 516, R. K. Razdan, Process for the preparation of (-)6a,10a,trans-6a,7,8,10a-tetrahydrodibenzo[b,d]-pyrans, Nr. 5 342 971, T. J. Herlt, Process for the Preparation of Dibenzo[b,d]-pyrans und Nr. 5 227 537, P. Stoss, Method for the production of 6,12-Dihydro-6-hydroxy-cannabidiol and the use thereof for the production of trans-delta-9-tetrahydrocannabinol) sind diese Synthesewege ausführlich dargestellt. Ferner sind in der deutschen Patentschrift DE 101 06 024 B4 von der THC Pharm GmbH, Verfahren zur Herstellung von Dronabinol, die Verwendung von Lewis-Säuren Katalysatoren beschrieben.

Nachteilig an den Δ⁹-THC-Sytheseverfahren des Standes der Technik ist, daß die Selektivität für Δ⁹-THC bei der Cylisierung von CBD immer noch zu gering ist und relativ viel Δ⁸-THC/iso-THC als unerwünschtes Nebenprodukt entsteht.

Ausgehend von dem oben erläuterten Stand der Technik sowie der neuen rechtlichen Situation in der Bundesrepublik Deutschland war es daher Aufgabe der vorliegenden Erfindung, ein Syntheseverfahren zur Herstellung von Δ⁹-THC durch Cyclisierung von CBD zur Verfügung zu stellen, bei dem die Selektivität für Δ⁹-THC gegenüber Δ⁸-THC/iso-THC verbessert ist.

Verfahrenstechnisch erfolgt die Lösung dieser Aufgabe durch die kennzeichnenden Merkmale des Patentanspruchs 1.

Überraschenderweise haben die Erfinder der vorliegenden Erfindung herausgefunden, dass der Einsatz eines Molekularsiebs in einem Verfahren zur Herstellung von Dronabinol ((6aR-trans)-6a, 7,8,10a-tetrahydro-6,6,9-trimethyl-3-pentyl-6H-dibenzo[b,d]pyran-1-ol, [delta] Δ⁹-Tetrahydrocannabinol (Δ⁹-THC)) durch Cyclisierung von Cannabidiol (CBD) (2-[1R-3-Methyl-6-(1-methylethenyl]-2-cyclohexen-1-yl]-5-pentyl-1,3-benzoldiol) in einem organischen Lösungsmittel einen starken Einfluss und auf die Selektivität in Bezug auf das Δ⁹-THC/Δ⁸-THC-Verhältnis und auf die Reaktionsgeschwindigkeit hat.

Im Stand der Technik ist, wie oben beschrieben, die Cyclisierung zu Δ⁹-THC in Gegenwart von Lewis-Säuren bekannt.

Die Erfinder haben überraschenderweise herausgefunden, dass wenn man die Lewis-Säure durch ein Molekularsieb ersetzt, die Δ⁹-THC-Ausbeute im Verhältnis zum gebildeten Δ⁸-THC deutlich steigt.

Die Cyclisierung wird durch die erfindungsgemäße Verwendung eines Molekularsiebes deutlich beschleunigt. Möglicherweise wirkt das Molekularsieb hierbei als Katalysator.

Als Molekularsieb kommen sämtliche im Stand der Technik bekannten Molekularsiebe in Betracht, insbesonder jedoch Molekularsiebe auf Basis von kristallinen Zeolith-Strukturen sowie Molekularsiebe aus synthetischen Zeolith-Analoga, wie sie beispielsweise in Kosal, M. E.; Chou, J.-H.; Wilson, S. R.; Suslick, K. S. "A Functional Zeolite Analogue Assembled From Metalloporphyrins" Nature Materials, 2002, 1, 118-121, offenbart sind, wobei diesbezüglich auf diese Druckschrift hiermit vollinhaltlich Bezug genommen wird.

Auch Mischungen unterschiedlicher Molekularsiebtypen sind anwendbar.

Für die Verwendung im Rahmen der vorliegenden Erfindung ist es bevorzugt, dass das Molekularsieb eine Porengröße von 0,2-1 nm, bevorzugt von 0,4 nm aufweist.

Eine kontinuierliche Erhöhung des Molekularsiebanteils bei dieser Umsetzung führt zu einer deutlichen Reaktionsbeschleunigung, was eine Reaktionszeitverkürzung von bis zu 50% zur Folge hat.

Vorteilhafterweise beträgt das Gewichtverhältnis Δ⁹-Tetrahydrocannabinol Cannabidiol (CBD)/Molekularsieb 5:1 bis 1:5, besonders bevorzugt 1:1.

Durch den Einsatz von einem Molekularsieb wird die Ausbeute an Δ⁹-THC erhöht, das Verhältnis von Δ⁹-THC zu Δ⁸-THC/iso-THC wird deutlich zu Gunsten des Δ⁹-THC verschoben, die Summe der Nebenprodukte nimmt deutlich ab und die Nebenprodukte als Folge des Abbauprozesses von Δ⁹-THC durch thermische Belastung werden verringert.

Die Erfinder haben weiterhin überraschenderweise herausgefunden, dass wenn ein Lewis-Säure Katalysator zusammen mit einem Molekularsieb verwendet wird, die Cyclisierungsreaktion stattfindet, schneller abläuft, bessere Ausbeuten an Δ⁹-THC und ein besseres Δ⁹-THC zu Δ⁸-THC/iso-THC - Verhältnis, verglichen mit einer Cyclisierungsreaktion mit einem Lewis-Säure Katalysator und ohne Molekularsieb, liefert.

Somit ist es in einer Ausführungsform der vorliegenden Erfindung vorgesehen, dass dem Umsetzungsgemisch zusätzlich eine Lewis-Säure zugesetzt wird. Beispielsweise kann diese Lewis-Säure Zinksalze, Zinnsalze, Magnesiumsalze und/oder Silbersalze, bevorzugt als Halogenide oder Trifluormethansulfonate umfassen. Besonders bevorzugt ist, dass Zinkbromid als Lewis-Säure verwendet wird.

Wird bei der Cyclisierungsumsetzung CBD mit einem Molekularsieb und einer Lewis-Säure umgesetzt, ist bevorzugt, dass das Gewichtsverhältnis CBD/Molekularsieb/Lewis-Säure zwischen 5:1:1, 1:5:1 und 1:1:5, bevorzugt bei 1:2:2 oder bei 1:2:3 und besonders bevorzugt bei 1:1:3 liegt, um ein besonders optimales Δ⁹-THC/Δ⁸-THC-Verhältnis zu erhalten.

Um möglichst hohe Ausbeuten an Δ⁹-THC zu erzielen, wird CBD mit Mischungen aus Lewis-Säuren und Molekularsieb umgesetzt, wobei der Anteil an Molekularsieb in der Mischung gleich oder erhöht, gegenüber dem Anteil der Lewis-Säure ist.

Zu hohe Anteile am Molekularsieb beschleunigen die Reaktionsgeschwindigkeit weiter, aber die Ausbeuten an Δ⁹-THC nehmen ab.

In einer Ausführungsform der vorliegenden Erfindung ist es bevorzugt, dass das in einem Lösungsmittel gelöste Cannabidiol mit dem Molekularsieb, gegebenenfalls in Gegenwart einer Lewis-Säure, unter Erhitzen, vorzugsweise unter Rückfluss, in Kontakt gebracht wird.

Um die Reaktionsgeschwindigkeit weiter zu beschleunigen ist es besonders bevorzugt, dass das Umsetzungsgemisch bis zu seinem Siedepunkt erhitzt wird.

Als mögliche Lösungsmittel kommen zunächst sämtliche organischen Lösungsmittel in Betracht, in denen Cannabidiol löslich ist.

Bevorzugt ist das Lösungsmittel ausgewählt aus der Gruppe bestehend aus aliphatischen Kohlenwasserstoffen insbesondere n-Pentan, n-Hexan, n-Heptan; aromatischen Kohlenwasserstoffen, insbesondere Benzol, Toluol, Xylol; halogenierten Kohlenwasserstoffen, insbesondere Dichlormethan, Dichlorethan, 1,1,1-Trichlorethan, Trichlorethen, Tetrachlorethen, Methylenchlorid; Petrolethern; cyclischen Aliphaten, insbesondere Cyclohexan und Mischungen daraus.

Ohne dass die Erfinder an diese Theorie gebunden sein wollen, wird vermutet, dass Wasserreste, die in den organischen Lösungsmitteln enthalten sein können, die Bildung des thermodynamisch stabileren Δ⁸-THC unterstützen.

Demnach ist es in einer Ausführungsform der vorliegenden Erfindung bevorzugt, dass das Lösungsmittel im Wesentlichen wasserfrei, besonders bevorzugt vorgetrocknet ist.

Es ist weiterhin vorteilhaft, die gesamte Umsetzung unter Schutzgasatmosphäre (beispielsweise N₂, Ar oder Mischungen daraus) auszuführen.

Eine solche Vortrocknung kann beispielsweise unter Verwendung von Natrium, Natrium-, Calcium-, Magnesiumsulfat und/oder einem Molekularsieb ausgeführt werden.

Auch ist bevorzugt, daß die optional zugesetzte Lewis-Säure im wesentlichen wasserfrei wird.

Nach erfolgreich abgeschlossener Cyclisierung von Cannabidiol (CBD) zu Dronabinol (Δ⁹-Tetrahydrocannabinol (Δ⁹-THC)) und dem Nebenprodukt Δ⁸-THC, kann das erwünschte Δ⁹-THC aus dem Δ⁹-THC/Δ⁸-THC-Gemisch beispielsweise über eine präparative HPLC-Säule mit modifiziertem Kieselgel, beispielsweise Typ C8 oder Typ C18, aufgereinigt werden.

Das Reaktionsgemisch aus der Cyclisierungsumsetzung wird hierzu in einem organischen Lösungsmittel oder Lösungsmittelgemisch gelöst.

In Betracht kommen unter anderem aliphatische Kohlenwasserstoffe insbesondere Pentan, Hexan, n-Hexan, n-Heptan; aromatische Kohlenwasserstoffe, insbesondere Benzol, Toluol, Xylol; halogenierte Kohlenwasserstoffe, insbesondere Dichlormethan, Dichlorethan, 1,1,1-Trichlorethan, Trichlorethen, Tetrachlorethen, Methylenchlorid; Petrolether; cyclische Aliphate, insbesondere Cyclohexan und Mischungen daraus.

Das Lösungsmittelgemisch mit dem darin enthaltenen Reaktionsgemisch wird auf die präparative HPLC-Säule aufgetragen.

Hierbei hängt das genaue Verfahren zur Trennung der Substanzen beispielsweise von der Art der Säule und von den verwendeten Lösungsmitteln ab. Es liegt im Bereich der Fähigkeit des Fachmanns, das jeweilige Trennverfahren optimal auf die jeweils vorliegenden Parameter anzupassen.

Nach erfolgter Trennung wird das erhaltene Dronabinol (Δ⁹-THC) vom Lösungsmittel befreit. Dies geschieht bevorzugt durch Destillation.

Überraschenderweise haben die vorliegenden Erfinder somit herausgefunden, dass bei dem erfindungsgemäßen Verfahren das Molekularsieb, neben den bisher beschriebenen trocknenden Eigenschaften, auch starke katalytische Eigenschaften besitzt, die bei dieser Umsetzung im Vordergrund stehen.

Cyclisierungen nur mit Lewis-Säure Katalysator sind in der Regel deutlich langsamer und liefern schlechtere Ausbeuten an Δ⁹-THC, als Cyclisierungen, die in Gegenwart eines Molekularsiebes durchgeführt werden.

Weitere Vorteile und Merkmale der vorliegenden Erfindung ergeben sich aufgrund der Beschreibung von Ausführungsbeispielen.

### Beispiel 1:

Zu einer Lösung von 0,5 g Cannabidiol (CBD) in 80 ml Heptan werden 0,5 g Molekularsieb mit einer Porengröße von 0,4 nm (Perlform, 2mm) gegeben. Die Lösung wird unter Rückfluss gekocht. Mittels HPLC Analytik werden die prozentualen Anteile der Komponenten nach 4 Stunden bestimmt.

Bei dieser Umsetzung von CBD nach Δ⁹-THC ohne Zinkbromid als Lewis-Säure und mit Molekularsieb, werden in den ersten vier Stunden Reaktionszeit ca. 28 % des Eduktes abgebaut. Mittels HPLC Analytik wurden der prozentualen Anteile von CBD (ca. 72 %), Δ⁹-THC (23,0 %) und Δ⁸-THC/iso-THC (3,5 %) bestimmt. Das Verhältnis von Δ⁹-THC zu Δ⁸-THC/iso-THC ist 6,6 : 1.

### Beispiel 2:

Zu einer Lösung von 0,5 g Cannabidiol (CBD) in 80 ml Heptan werden 0,5 g Zinkbromid gegeben. Die Lösung wird unter Rückfluss gekocht. Mittels HPLC Analytik werden die prozentualen Anteile der Komponenten nach 4 Stunden bestimmt.

Bei der Umsetzung von CBD nach Δ⁹-THC mit Zinkbromid als Lewis-Säure und ohne Molekularsieb, werden in den ersten vier Stunden Reaktionszeit ca. 11 % des Eduktes abgebaut. Mittels HPLC Analytik wurden der prozentualen Anteile von CBD (ca. 89 %), Δ⁹-THC (ca.6,5 %) und Δ⁸-THC/iso-THC (ca. 2,0 %) bestimmt. Das Verhältnis von Δ⁹-THC zu Δ⁸-THC/iso-THC ist 3,2 : 1.

### Beispiel 3:

Zu einer Lösung von 0,5 g Cannabidiol (CBD) in 80 ml Heptan werden 0,5 g Zinkbromid gegeben. Die Menge des Molekularsiebes (Porengröße 0,4 nm) wird variiert, von 0,5 g bis 2,5 g. Die Lösung wird unter Rückfluss gekocht. Mittels HPLC Analytik werden die prozentualen Anteile der Komponenten nach 4 und nach 7 Stunden bestimmt.
a) prozentuale Anteile nach 4 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 81,7 | 14,1 | 1,8 | 7,8 | 1 | 1 | 1 |
| 72,2 | 22,2 | 3,6 | 6,1 | 1 | 2 | 1 |
| 46,8 | 44,5 | 6,0 | 7,4 | 1 | 3 | 1 |
| 32,6 | 54,6 | 9,4 | 5,8 | 1 | 5 | 1 |

b) prozentuale Anteile nach 7 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 69.5 | 25,9 | 3,0 | 8,6 | 1 | 1 | 1 |
| 56,2 | 35,4 | 5,2 | 6,8 | 1 | 2 | 1 |
| 29,9 | 60,5 | 8,5 | 7,1 | 1 | 3 | 1 |
| 15,9 | 66,8 | 12,9 | 5,2 | 1 | 5 | 1 |

### Beispiel 4:

Zu einer Lösung von 0,5 g Cannabidiol (CBD) in 80 ml Heptan werden 1,0 g Zinkbromid gegeben. Die Menge des Molekularsiebes (Porengröße 0,4 nm) wird variiert, von 0,5 g bis 2,5 g. Die Lösung wird unter Rückfluss gekocht. Mittels HPLC Analytik werden die prozentualen Anteile der Komponenten nach 4 und nach 7 Stunden bestimmt.
a) prozentuale Anteile nach 4 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 81,7 | 14,9 | 2,1 | 7,1 | 1 | 1 | 2 |
| 55,0 | 38,4 | 4,2 | 9,1 | 1 | 2 | 2 |
| 51,3 | 40,3 | 5,5 | 7,3 | 1 | 3 | 2 |
| 27,4 | 60,2 | 9,8 | 6,1 | 1 | 5 | 2 |

b) prozentuale Anteile nach 7 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 66,7 | 28,6 | 3,1 | 9,2 | 1 | 1 | 2 |
| 26,4 | 64,4 | 6,7 | 9,6 | 1 | 2 | 2 |
| 25,2 | 63,3 | 9,1 | 6,9 | 1 | 3 | 2 |
| 5,8 | 75,8 | 15,1 | 5,0 | 1 | 5 | 2 |

### Beispiel 5:

Zu einer Lösung von 0,5 g Cannabidiol (CBD) in 80 ml Heptan werden 1,5 g Zinkbromid gegeben. Die Menge des Molekularsiebes (Porengröße 0,4 nm) wird variiert, von 0,5 g bis 2,5 g. Die Lösung wird unter Rückfluss gekocht. Mittels HPLC Analytik werden die prozentualen Anteile der Komponenten nach 4 und nach 7 Stunden bestimmt.
a) prozentuale Anteile nach 4 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 82,8 | 13,7 | 1,8 | 7,4 | 1 | 1 | 3 |
| 59,0 | 35,2 | 4,0 | 8,8 | 1 | 2 | 3 |
| 41,7 | 49,0 | 6,2 | 7,9 | 1 | 3 | 3 |
| 28,2 | 59,1 | 10,1 | 5,8 | 1 | 5 | 3 |

b) prozentuale Anteile nach 7 Stunden

| Anteil CBD [%] | Anteil Δ⁹-THC [%] | Anteil Δ⁸-THC [%] | Δ⁹-THC/ Δ⁸-THC | Gewichtsteile | | |
|---|---|---|---|---|---|---|
| | | | | CBD | Molekularsieb | Zinkbromid |
| 69,5 | 26,5 | 2,7 | 9,8 | 1 | 1 | 3 |
| 32,0 | 59,6 | 6,9 | 9,7 | 1 | 2 | 3 |
| 14,0 | 73,0 | 9,1 | 7,7 | 1 | 3 | 3 |
| 10,3 | 72,2 | 14,4 | 5,0 | 1 | 5 | 3 |

## Patentansprüche

1. Verfahren zur Herstellung von Dronabinol (A) ((6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹THC)) aus Cannabidiol (CBD) (B) ((2-[1R-3-Methyl-6-(1-methylethenyl]-2-cyclohexen-1-yl]-5-pentyl-1,3-benzoldiol)) 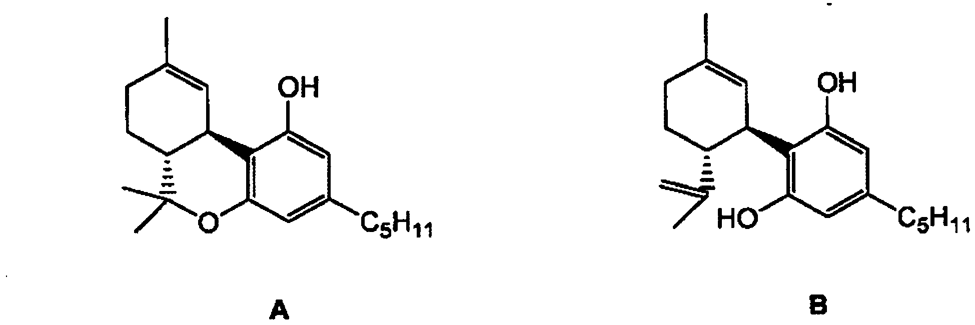 **dadurch gekennzeichnet,**
**dass** Cannabidiol (CBD) (B) in einem organischen Lösungsmittel vorgelegt und in Gegenwart eines Molekularsiebs als Katalysator unter Erhitzen zu Δ⁹-THC cyclisiert wird.

2. Verfahren nach Anspruch 1, wobei das Molekularsieb eine Porengröße von 0,2 - 1 Nanometer (2-10 Å) aufweist.

3. Verfahren nach Anspruch 1 oder 2, wobei das Molekularsieb eine Porengröße von 0,4 - 1 Nanometer (4-10 Å) aufweist.

4. Verfahren nach einem der Ansprüche 1-3, **dadurch gekennzeichnet, dass** das in einem Lösungsmittel gelöste Cannabidiol mit dem Molekularsieb unter Erhitzen unter Rückfluss in Kontakt gebracht wird.

5. Verfahren nach einem der Ansprüche 1-4, **dadurch gekennzeichnet, dass** das Cannabidiol-Lösungsmittelgemisch bis zum Siedepunkt des Umsetzungsgemisches, umfassend Molekularsieb und Cannabidiol gemäß Anspruch 1, erhitzt wird.

6. Verfahren nach einem der Ansprüche 1-5, **dadurch gekennzeichnet, dass** das Lösungsmittel im Wesentlichen wasserfrei ist.

7. Verfahren nach einem der Ansprüche 1-6, **dadurch gekennzeichnet, dass** das Lösungsmittel ausgewählt ist aus aliphatischen Kohlenwasserstoffen insbesondere Pentan, Hexan, n-Hexan, n-Heptan, aromatischen Kohlenwasserstoffen, insbesondere Benzol, Toluol, Xylol, halogenierten Kohlenwasserstoffen, insbesondere Dichlormethan, Dichlorethan, 1,1,1-Trichlorethan, Trichlorethen, Tetrachlorethen, Methylenchlorid, Petrolethern, cyclischen Aliphaten, insbesondere Cyclohexan und Mischungen daraus.

8. Verfahren nach einem der Ansprüche 1-6, **dadurch gekennzeichnet, dass** das Lösungsmittel vorgetrocknet wird.

9. Verfahren gemäß Anspruch 8, **dadurch gekennzeichnet, dass** die Vortrocknung unter Verwendung von Natrium, Natrium-, Calcium-, Magnesiumsulfat und/oder einem Molekularsieb ausgeführt wird.

10. Verfahren nach einem der Ansprüche 1-9, **dadurch gekennzeichnet, dass** die Cyclisierung unter Schutzgas, bevorzugt N₂, Ar oder Mischungen daraus, ausgeführt wird.

11. Verfahren nach einem der Ansprüche 1-10, **dadurch gekennzeichnet, dass** dem Umsetzungsgemisch zusätzlich Lewis-Säure, bevorzugt eine im Wesentlichen wasserfreie Lewis-Säure zugesetzt wird.

12. Verfahren gemäß Anspruch 11, **dadurch gekennzeichnet, dass** die Lewis-Säure Zinksalze, Zinnsalze, Magnesiumsalze und/oder Silbersalze, bevorzugt als Halogenide oder Trifluormethansulfonate umfasst.

13. Verfahren gemäß Anspruch 11 oder 12, **dadurch gekennzeichnet, dass** die Lewis-Säure Zinkbromid ist.

14. Verfahren nach einem der Ansprüche 1-13, **dadurch gekennzeichnet, dass** das Gewichtverhältnis Cannabidiol (2)/Molekularsieb 5:1 bis 1:5, bevorzugt 1:1 beträgt.

15. Verfahren nach einem der Ansprüche 1-14, **dadurch gekennzeichnet, dass** das Gewichtsverhältnis Cannabidiol (2)/Molekularsieb/Lewis-Säure zwischen 5:1:1, 1:5:1 und 1:1:5, bevorzugt bei 1:2:2 oder bei 1:2:3 liegt.

16. Verfahren gemäß Anspruch 15, **dadurch gekennzeichnet, dass** der Anteil an Molekularsieb in der Mischung gleich oder erhöht, gegenüber dem Anteil der Lewis-Säure ist.

17. Verfahren nach einem der Ansprüche 1-16, **dadurch gekennzeichnet, dass** das Molekularsieb ausgewählt ist aus Molekularsieben auf Basis von kristallinen Zeolith-Strukturen oder Molekularsieben auf Basis von synthetischen Zeolith-Analoga.

## Claims

1. A method for producing dronabinol (A) ((6aR-trans)-6a,7,8,10a-tetrahydro-6,6,9-trimethyl-3-pentyl-6H-dibenzo[b,d]pyran-1-ol, Δ⁹-tetrahydrocannabinol (Δ⁹THC)) from cannabidiol (CBD) (B) ((2-[1R-3-methyl-6-(1-methylethenyl]-2-cyclohexen-1-yl]-5-pentyl-1,3-benzenediol)) 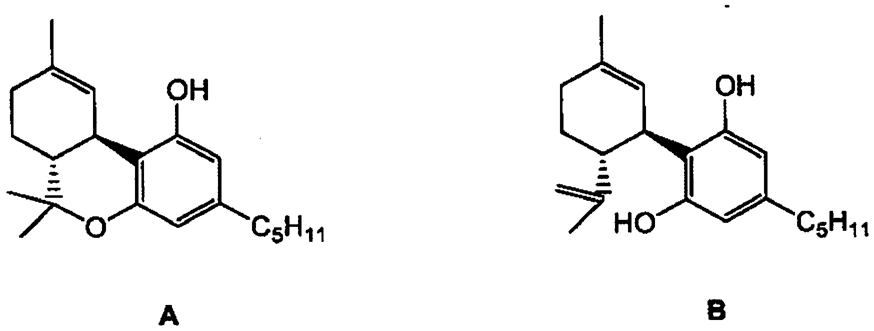 **characterised in that**
cannabidiol (CBD) (B) is placed in an organic solvent and is cyclised in the presence of a molecular sieve as catalyst under heating to form Δ⁹-THC.

2. The method according to Claim 1, wherein the molecular sieve has a pore size of 0.2 - 1 nanometres (2-10 Å).

3. The method according to Claim 1 or 2, wherein the molecular sieve has a pore size of 0.4 - 1 nanometres (4-10 A).

4. The method according to one of Claims 1-3, **characterised in that** the cannabidiol dissolved in a solvent is brought into contact with the molecular sieve under heating and under reflux.

5. The method according to one of Claims 1-4, **characterised in that** the cannabidiol solvent mixture is heated to the boiling point of the conversion mixture, comprising molecular sieve and cannabidiol according to Claim 1.

6. The method according to one of Claims 1-5, **characterised in that** the solvent is substantially free from water.

7. The method according to one of Claims 1-6, **characterised in that** the solvent is selected from aliphatic hydrocarbons, in particular pentane, hexane, n-hexane, and n-heptane, aromatic hydrocarbons, in particular benzene, toluene, and xylene, halogenated hydrocarbons, in particular dichloromethane, dichloroethane, 1,1,1-trichloroethane, trichloroethene, and tetrachloroethene, methylene chloride, petroleum ethers, and cyclic aliphatics, in particular cyclohexane, and mixtures thereof.

8. The method according to one of Claims 1-6, **characterised in that** the solvent is predried.

9. The method according to Claim 8, **characterised in that** the predrying is performed with use of sodium, sodium sulphate, calcium sulphate, magnesium sulphate and/or a molecular sieve.

10. The method according to one of Claims 1-9, **characterised in that** the cyclisation is performed under protective gas, preferably N₂, Ar or mixtures thereof.

11. The method according to one of Claims 1-10, **characterised in that** Lewis acid, preferably a substantially anhydrous Lewis acid, is additionally added to the conversion mixture.

12. The method according to Claim 11, **characterised in that** the Lewis acid comprises zinc salts, tin salts, magnesium salts and/or silver salts, preferably as halides or trifluoromethanesulfonates.

13. The method according to Claim 11 or 12, **characterised in that** the Lewis acid is zinc bromide.

14. The method according to one of Claims 1-13, **characterised in that** the ratio by weight of cannabidiol (2)/molecular sieve is 5:1 to 1:5, preferably 1:1.

15. The method according to one of Claims 1-14, **characterised in that** the ratio by weight of cannabidiol (2)/molecular sieve/Lewis acid is between 5:1:1, 1:5:1 and 1:1:5, and preferably is 1:2:2 or is 1:2:3.

16. The method according to Claim 15, **characterised in that** the proportion of molecular sieve in the mixture is equal to or greater than the proportion of Lewis acid.

17. The method according to one of Claims 1-16, **characterised in that** the molecular sieve is selected from molecular sieves based on crystalline zeolite structures or molecular sieves based on synthetic zeolite analogues.

## Revendications

1. Procédé de fabrication de dronabinol (A) ((6aR-trans)-6a,7,8,10a-tétrahydro-6,6,9-triméthyl-3-pentyl-6H-dibenzo[b,d]pyran-1-ol, Δ⁹-tétrahydrocannabinol (Δ⁹THC)) à partir de cannabidiol (CBD) (B) ((2-[1R-3-méthyl-6-(1-méthyléthényl]-2-cyclohèxen-1-yl]-5-pentyl-1,3-benzoldiol)) 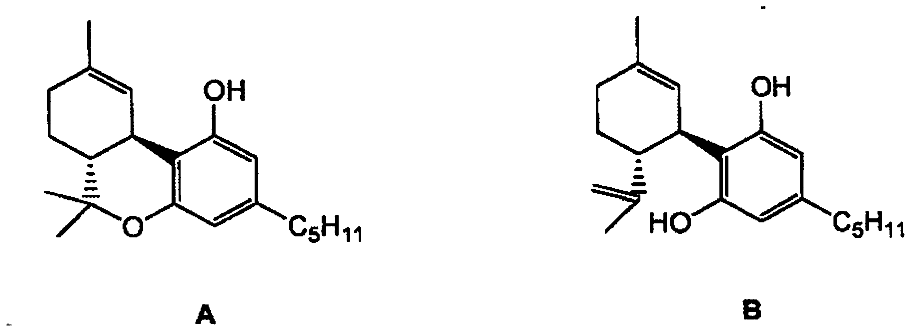 **caractérisé en ce**
**que** le cannabidiol (CBD) (B) est présent en milieu solvant organique et est cyclisé en Δ⁹-THC par un chauffage en présence d'un tamis moléculaire en tant que catalyseur.

2. Procédé selon la revendication 1, dans lequel le tamis moléculaire possède une taille des pores de 0,2 à 1 nanomètres (2 à 10 Å).

3. Procédé selon les revendications 1 ou 2, dans lequel le tamis moléculaire possède une taille des pores de 0,4 à 1 nanomètres (4 à 10 Å).

4. Procédé selon l'une des revendications 1 à 3, **caractérisé en ce que** le cannabidiol en solution dans un solvant est mis en contact avec le tamis moléculaire avec un chauffage sous reflux.

5. Procédé selon l'une des revendications 1 à 4, **caractérisé en ce que** le mélange cannabidiol-solvant est réchauffé jusqu'au point d'ébullition du mélange réactionnel comprenant le tamis moléculaire et le cannabidiol selon la revendication 1.

6. Procédé selon l'une des revendications 1 à 5, **caractérisé en ce que** le solvant est essentiellement exempt d'eau.

7. Procédé selon l'une des revendications 1 à 6, **caractérisé en ce que** le solvant est choisi parmi les hydrocarbures aliphatiques , notamment, le pentane, l'hexane, le n-hexane, le n-heptane, les hydrocarbures aromatiques, notamment, le benzène, le toluène, le xylène, les hydrocarbures halogénés, notamment, le dichlorométhane, le dichloroéthane, le 1,1,1-trichloroéthane, le trichloroéthène, le tétrachloroéthène, le chlorure de méthylène, l'éther de pétrole, les aliphatiques cycliques, notamment, le cyclohexane et des mélanges de ceux-ci.

8. Procédé selon l'une des revendications 1 à 6, **caractérisé en ce que** le solvant est préalablement séché.

9. Procédé selon la revendication 8, **caractérisé en ce que** le séchage préalable est effectué moyennant l'utilisation de sodium, de sulfate de sodium, de sulfate de calcium, de sulfate de magnésium et/ou d'un tamis moléculaire.

10. Procédé selon l'une des revendications 1 à 9, **caractérisé en ce que** la cyclisation est effectuée sous l'atmosphère d'un gaz protecteur, de préférence, N₂, Ar ou des mélanges de ceux-ci.

11. Procédé selon l'une des revendications 1 à 10, **caractérisé en ce qu'**un acide de Lewis, de préférence, un acide de Lewis essentiellement exempt d'eau, est ajouté de manière complémentaire au mélange réactionnel.

12. Procédé selon la revendication 11, **caractérisé en ce que** l'acide de Lewis comprend des sels de zinc, des sels d'étain, des sels de magnésium et/ou des sels d'argent, de préférence sous formes d'halogénures ou de trifluorométhanesulfonates.

13. Procédé selon les revendications 11 ou 12, **caractérisé en ce que** l'acide de Lewis est du bromure de zinc.

14. Procédé selon l'une des revendications 1 à 13, **caractérisé en ce que** le rapport en poids cannabidiol (2)/tamis moléculaire s'élève de 5 : 1 à 1 : 5, est de préférence de 1 : 1.

15. Procédé selon l'une des revendications 1 à 14, **caractérisé en ce que** le rapport en poids cannabidiol (2)/tamis moléculaire/acide de Lewis se situe parmi 5 : 1 : 1, 1 : 5 : 1 et 1 : 1 : 5, de préférence à 1 : 2 : 2 ou à 1 : 2 : 3.

16. Procédé selon la revendication 15, **caractérisé en ce que** la proportion de tamis moléculaire dans le mélange est égale, ou supérieure, par rapport à la proportion d'acide de Lewis dans le mélange.

17. Procédé selon l'une des revendications 1 à 16, **caractérisé en ce que** le tamis moléculaire est choisi sur la base de structures de zéolithes cristallines ou de tamis moléculaires sur la base d'analogues de zéolithes synthétiques.
